# EUROPÄISCHE PATENTSCHRIFT

(11) **EP 0 904 355 B1**
(45) Veröffentlichungstag und Bekanntmachung des Hinweises auf die Patenterteilung: **03.03.2004**
(21) Anmeldenummer: 97928173.0
(22) Anmeldetag: 11.06.1997
(51) Int. Cl.: C12N 9/00

(54) **VERFAHREN ZUR AKTIVIERUNG VON DENATURIERTEM PROTEIN**
METHOD OF ACTIVATING DENATURED PROTEIN
PROCEDE D'ACTIVATION DE PROTEINE DENATUREE

(30) Priorität: 11.06.1996 EP 96109288; 22.06.1996 EP 96110109
(43) Veröffentlichungstag der Anmeldung: 31.03.1999
(73) Patentinhaber: Roche Diagnostics GmbH, 68305 Mannheim (DE)
(72) Erfinder: GROSSMANN, Adelbert, D-82436 Eglfing (DE)
(74) Vertreter: Schreiner, Siegfried, Dr.
(86) Internationale Anmeldenummer: PCT/EP1997/003026
(87) Internationale Veröffentlichungsnummer: WO 1997/047735

(56) Entgegenhaltungen:
- EP-A- 0 114 506
- EP-A- 0 361 475
- WO-A-87/02673
- C. WHITE ET AL.: "Investigation of the redox state of recombinant horseradish peroxidase produced in inclusion bodies and factors affecting the efficiency of refolding" BIOCHEMICAL SOCIETY TRANSACTIONS, Bd. 23, 1995, Seite 138S XP000604007
- B. FISCHER ET AL.: "Isolation, renaturation and formation of disulfide bonds of eukaryotic proteins expressed in E. coli as inclusion bodies" BIOTECHNOLOGY AND BIOENGINEERING, Bd. 41, 1993, Seiten 3-13, XP000605146
- YAN-NA ZHANG AND R.D. GRAY: "Characterization of folded, intermediate and unfolded states of recombinant human interstitial collagenase" JOURNAL OF BIOLOGICAL CHEMISTRY, Bd. 271, Nr. 14, 1996, Seiten 8015-8021, XP000605153

## Beschreibung

Die vorliegende Erfindung betrifft ein vereinfachtes Verfahren zur Solubilisierung und Naturierung von denaturierten Proteinen, insbesondere von rekombinant hergestellten denaturierten Proteinen.

Bei der Herstellung von Proteinen in prokaryontischen Zellen, wie z. B. E.coli, entstehen häufig schwerlösliche inaktive Proteinaggregate (inclusion bodies). Um diese Proteine in ihre aktive Form zu überführen, ist es erforderlich, diese Proteine zu solubilisieren und zu naturieren. Derartige Verfahren sind bekannt und beispielsweise in EP-A 0 361 475, EP-A 0 114 506, EP-A 0 093 619, EP-A 0 253 823, WO 87/02673, EP-A 0 364 926 und EP-A 0 241 022 beschrieben. Ein wesentlicher, die Ausbeute an naturiertem Protein limitierender Faktor bei der Aktivierung ist die Konkurrenzreaktion zwischen einer Überführung des naturierten Proteins in das richtige Faltungsintermediat und einer Aggregation von mehreren Proteinmolekülen. Aus diesem Grund ist die Konzentration an naturiertem Protein in der Naturierungslösung ein wesentlicher Parameter für die Ausbeute des Naturierungsverfahrens. Mit steigender Konzentration an naturiertem Protein wird die Aggregation begünstigt und die relative Ausbeute an naturiertem Protein mit der Konformation des nativen Proteins sinkt (kritische Konzentration).

Bei einer großtechnischen Herstellung von rekombinanten Proteinen ist die Menge an zu naturierendem Protein üblicherweise wesentlich höher als die kritische Konzentration. Bei der oft geringen Löslichkeit der Proteine im verwendeten Aktivierungspuffer, ergeben sich daher erhebliche Nachteile, wie geringe Ausbeute, hoher Zeitbedarf und große Puffervolumina.

Aus der WO 87/02673 ist ein Verfahren bekannt, bei dem das inaktive lösliche Protein mit Denaturierungsmittel und Reduktionsmittel solubilisiert wird, anschließend das Reduktionsmittel abgetrennt wird und dann von den solubilisierten Proteinen heterologe gemischte Disulfide zwischen Protein und beispielsweise Glutathion hergestellt werden. Derartige gemischte Disulfide sind für die weitere Reinigung und Renaturierung vorteilhaft, da nach Modifizierung der Thiolgruppen das Protein gegen Luftoxidation geschützt ist und es damit in einem größeren pH-Bereich stabil ist. Ebenso erleichtert die Veränderung der Nettoladung die Reinigung, da damit über eine Ionenaustauschchromatographie nichtmodifizierte Proteine abgetrennt werden können.

Zur Bildung der gemischten Disulfide wird das solubilisierte, dialysierte, reduzierte und von Reduktionsmitteln gereinigte Protein mit einer Lösung, welche ein Denaturierungsmittel und eine Disulfidkomponente zur Derivatisierung (z. B. GSSG, Cystin, Cystamin) enthält, inkubiert. Nach Abtrennung der Disulfidkomponente, wird eine Naturierung in üblicher Weise durchgeführt. Dieses Verfahren ist zwar effizient, erfordert jedoch die Durchführung einer Vielzahl von einzelnen Verfahrensschritten, insbesondere die Abtrennung des Reduktionsmittels vor der Derivatisierung.

Aus der WO 93/19084 ist ein Verfahren zur Faltung und Reinigung von Insulin-Like-Growth-Factor I bekannt. Danach werden die inclusion bodies unter reduzierenden Bedingungen gelöst, anschließend wird ein Überschuß an Oxidationsmittel zugegeben, ohne daß das Reduktionsmittel abgetrennt wird. Durch anschließende Verdünnung (ohne Dialyse) und erneute Zugabe von Reduktionsmittel (zum Aufbau eines Redox-Systems) wird die Naturierung eingeleitet. Mit der WO 91/08762 wird die Herstellung von biologisch aktivem plateletderived growth factor beschrieben. Hierbei erfolgt zunächst eine Solubilisierung bei pH 3 ohne Reduktionsmittel und anschließend eine Reinigung unter denaturierenden Bedingungen. Erst danach wird ein Oxidationsmittel zur Herstellung eines Derivats zugegeben. Gemäß der EP-A 0 450 386 wird ein Extrakt von inclusion bodies (denaturiert gelöstes NGF-Protein) durch Zugabe solubilisierender Puffer mit anschließender Zentrifugation hergestellt. Der Extrakt wird dann mit Reduktionsmittel behandelt, inkubiert und ohne vorhergehende Dialyse, durch Zugabe von Oxidationsmittel, oxidiert. Anschließend erfolgt eine Verdünnung und Zugabe weiterer Komponenten zur Naturierung. Bei diesem Verfahren wird also zunächst eine Solubilisierung, ohne Zugabe von Redox-aktiven Substanzen, als separater Verfahrensschritt durchgeführt. Keines dieser Verfahren ist für eine Pulsrenaturierung gemäß US-Patent Nr. 4,933,434 geeignet.

Weiter sind Verfahren bekannt, die eine Derivatisierung bereits bei der Solubilisierung ermöglichen. Seit langem bekannt ist die Methode der Sulfitolyse (z.B. Bailey, J.L., Cole, R.D., 1959, J. Biol. Chem. 234, 1733-1739; Cole, R.D., 1967, in: Meth. Enzymol. 11, 206-208; EP 0 114 507). Hierbei werden Disulfidbrücken in Proteinen mit Salzen der schwefligen Säure behandelt, wobei als Reaktionsprodukt ein Gemisch aus je 50% thiosulfonierten (RS-SO⁻₃) und freien (RS⁻) Protein-SH-Gruppen entsteht. Letztere freien SH-Gruppen werden durch Reoxidation (z.B. mit Kupfer-Ionen, Iodosobenzoat, oder bevorzugt mit Tetrathionat) wieder in Disulfide überführt, die durch erneute Zyklen des Prozesses weitgehend vollständig in das Thiosulfonat überführt werden. Dieser Prozeß ist relativ einfach und unter schonenden Randbedingungen (z.B. neutraler pH-Wert) durchzuführen. Nachteilig ist, wie bereits in J. Biol. Chem. 234, 1733 ausgeführt wird, daß das entstehende Thiosulfonat chemisch labil ist, die Vollständigkeit der Umsetzung nicht geprüft werden kann und vor allem durch die Reoxidationsmittel eine partielle Zerstörung der Tryptophanreste erfolgt. Weiterhin nachteilig ist, daß eine vollständige Abtrennung von Nebenprodukten mit thiosulfonierten Protein-SH-Gruppen sowie von Oxidationsmitteln wie dem genannten Iodosobenzoat im Endprodukt nur sehr schwer zu erreichen ist und analytisch nur mit erheblichem Aufwand nachgewiesen werden kann. Dies ist bei Proteinen, die therapeutisch eingesetzt werden sollen, aber unbedingt erforderlich, um mögliche Nebenwirkungen eines chemisch derart unphysiologisch veränderten Therapeutikums ausschließen zu können.

Eine derartige Sulfitolyse wird auch zur Naturierung von neurotrophen Faktoren der NGF/BDNF-Familie in der WO 95/30686 beschrieben. Ein analoges Verfahren wird für die Naturierung von humanem Proinsulin von R Wetzel et al., Gene 16 (1981) 63 - 71 sowie von W.F. Heath et al., J. Biol. Chem. 267 (1992) 419 - 425 beschrieben.

Eine ähnliche Methode, die die Verwendung von Seitenketten-schädigenden Reoxidationsbedingungen vermeidet, ist ebenfalls bekannt (Thannhauser, T.W., Konishi, Y., Scheraga, H.A., 1984, Analyt. Biochem 138, 181-188; Thannhauser, T.W., Scheraga, H.A., 1985, Biochemistry 24, 7681-7688): Hier wird statt einer Reoxidation die direkte Derivatisierung des bei der Reduktion der Disulfidbrücke erhaltenen Cysteins durch Reaktion mit 2-Nitro-5-(sulfothio)-benzoat bewirkt; hierbei wird 2-Nitro-5-thiobenzoat freigesetzt, das photometrisch gemessen werden kann und somit eine Quantifizierung der umgesetzen SH-Gruppen ermöglicht. Nachteilig an dieser Methode ist, daß hier eine komplexe chemische Substanz eingebracht wird, deren vollständige Abtrennung aus dem Endprodukt nur mit großem Aufwand realisiert und nachgeprüft werden kann. Weiterhin wurde von den Autoren (Biochemistry 24, 7681) beobachtet, daß das erhaltene Thiosulfonat nur bei vollständiger Abwesenheit von Thiolgruppen stabil ist. Außerdem wird auch hier eine Seitenkettenmodifikation beobachtet (Deamidierung von Asparagin).

Aufgabe der vorliegenden Erfindung ist es, diese Verfahren zu vereinfachen und zu verbessern sowie stabil lagerfähige an SH-Gruppen derivatisierte Proteine bereitzustellen, die mit hoher Ausbeute naturiert werden können.

Überraschenderweise wurde gefunden, daß nach dem erfindungsgemäßen Verfahren Solubilisierung und Derivatisierung in einem einzigen Schritt durchgeführt werden können, ohne daß zuvor reduziert werden muß. Besonders überraschend ist, daß die Derivatisierung auch unter sauren Bedingungen (pH-Wert kleiner als 7,0, vorzugsweise pH 3 - 6,5), vorzugsweise für Neurotrophine, wie NGF, und dies sogar ohne wesentlichen Einfluß auf Kinetik und Vollständigkeit der Reaktion im Vergleich zu einer Reaktion mit Thiol-Komponenten im üblichen pH-Bereich von etwa 7 - 10 stattfinden kann. Üblicherweise wurde angenommen, daß derartige Reaktionen nur in Gegenwart des freien Thiolat-Anions ablaufen können; dieses tritt aufgrund des hohen pK-Wertes von Thiolat-Anionen von etwa 9 in wirksamer Konzentration erst bei pH-Werten ab etwa 7 auf.

Gegenstand der Erfindung ist daher ein Verfahren zur Herstellung von gemischten Disulfiden aus einem Protein und einer Disulfidkomponente, welches dadurch gekennzeichnet ist, daß das Protein in einer inaktiven schwerlöslichen Form (inclusion bodies) mit einer Lösung eines Denaturierungsmittels in einer denaturierenden Konzentration und in Gegenwart einer Disulfidkomponente (molares Verhältnis Protein : Disulfidkomponente 1 : 1 bis 1 : 10000, vorzugsweise bis 1 : 1000) inkubiert, gelöst, derivatisiert und anschließend gegebenenfalls die Disulfidkomponente entfernt wird. Die Entfernung der Disulfidkomponente kann dann zweckmäßig durchgeführt werden, wenn anschließend eine Pulsrenaturierung erfolgt, wie sie in dem US-Patent Nr. 4,933,434 beschrieben ist. Das erfindungsgemäße derivatisierte Protein ist stabil und kann vor weiterer Verarbeitung gelagert werden. Dies ist besonders vorteilhaft, da unter Anwendung des erfindungsgemäßen Verfahrens die Herstellung des derivatisierten Proteins von der Naturierung unabhängig erfolgen kann. Auf diese Weise steht das derivatisierte Protein als isoliertes Zwischenprodukt für eine Mehrzahl von Naturierungs- und Aufreinigungsverfahren und/oder Ansätzen zur Verfügung.

Alternativ wird die Inkubation zur Derivatisierung des Proteins in Gegenwart eines Reduktionsmittels (z.B. DTT, DTE, GSH, Cystein, Cysteamin, Salze der schwefligen Säure) durchgeführt. Dadurch kann die Derivatisierungsausbeute verbessert werden. Die Konzentration des Reduktionsmittels wird dabei zweckmäßig so gewählt, daß die Wirksamkeit der Disulfidkomponente nicht oder nur geringfügig eingeschränkt wird; als günstig haben sich Reduktionsmittelkonzentrationen bis zu 20 Mol-%, vorzugsweise bis zu 10 % der Konzentration der Disulfidkomponente herausgestellt.

Zum Schutz freier SH-Gruppen können vorzugsweise weitere Reagentien zugesetzt werden, die eine Blockierung oder Zerstörung dieser SH-Gruppen durch Schwermetalle, Radikale oder aktive Sauerstoff spezies teilweise oder vollständig verhindern können. Zur Klasse dieser Schutzreagentien gehört beispielsweise EDTA in einer Konzentration von 0,1 bis 100 mmol/l oder Mannit in einer Konzentration von 1 bis 1000 mmol/l.

Unter Disulfidkomponente sind Substanzen aus der Klasse der Disulfide, z.B. GSSG, Cystamin oder Cystin, zu verstehen. Disulfidkomponenten sind in der Lage, nach Spaltung einer Disulfidbrücke in Proteinen SH-Gruppen zu derivatisieren. Die Disulfidkomponente wird vorzugsweise in einer Konzentration von mindestens 1 mmol/l oder höher, vorzugsweise von 1 - 1000 mmol/l, besonders bevorzugt von 10 - 200 mmol/l verwendet.

Als Denaturierungsmittel wird zweckmäßig ein für die Solubilisierung von denaturiertem Protein unter oxidierenden Bedingungen üblicherweise verwendetes Denaturierungsmittel eingesetzt. Bevorzugt werden Guanidiniumhydrochlorid oder andere Guanidiniumsalze, wie z. B. Sulfat, Phosphat oder Thiocyanat, sowie Harnstoff oder dessen Derivate verwendet. Ferner können Gemische dieser Denaturierungsmittel verwendet werden.

Die Konzentration des Denaturierungsmittels ist von der Art des Denaturierungsmittels abhängig und für einen Fachmann ohne weiteres feststellbar. Die Konzentration des Denaturierungsmittels ist dann ausreichend, wenn eine vollständige Solubilisierung des denaturierten schwerlöslichen Proteins erreicht werden kann. Für Guanidinhydrochlorid liegen diese Konzentrationen üblicherweise bei 3 - 8 mol/l, vorzugsweise 6 - 8 mol/l. Für Harnstoff liegen die Konzentrationen üblicherweise bei 6 - 10 mol/l.

Unter "Protein in einer inaktiven schwerlöslichen Form" ist ein Protein zu verstehen, welches beispielsweise nach rekombinanter Herstellung in Prokaryonten entsteht. Solche Proteine entstehen üblicherweise dann, wenn eine Überexpression eukaryontischer Proteine in Prokaryonten erfolgt und das Protein nicht in aktiver Form ins Periplasma oder in den Zellüberstand ausgeschleust wird. Dabei verbleibt das rekombinant hergestellte Protein in unlöslicher und aggregierter Form im Cytoplasma oder Periplasma. Derartige Aggregate, deren Isolierung und Reinigung sind beispielsweise in Marston F.A.O., Biochem. J. 214 (1986) 1 - 12 beschrieben. Zur Gewinnung von inclusion bodies werden nach der Fermentation die prokaryontischen Zellen aufgeschlossen.

Der Zellaufschluß kann durch übliche Methoden durchgeführt werden, z. B. mittels Ultraschall, Hochdruckdispersion oder Lysozym. Er wird vorzugsweise in einer zur Einstellung eines neutralen bis schwach sauren pH-Werts geeigneten Pufferlösung als Suspensionsmedium durchgeführt, wie z. B. 0,1 mol/l Tris-HCl. Nach ZeHaufschluß werden die unlöslichen Bestandteile (inclusion bodies) in beliebiger Weise abgetrennt, vorzugsweise durch Abzentrifugieren oder durch Filtration nach dem Waschen mit Agentien, die die Proteine nicht stören, fremde Zellproteine jedoch möglichst lösen, z. B. Wasser oder Phosphatpuffer, ggf. unter Zusatz milder Detergentien, wie Brij®. Anschließend wird der Niederschlag (Pellet) dem erfindungsgemäßen Verfahren zur Solubilisierung und Derivatisierung unterworfen.

Das erfindungsgemäße Verfahren wird im neutralen bis alkalischen pH-Bereich durchgeführt, vorzugsweise zwischen pH 6 und 10, besonders bevorzugt im pH-Bereich zwischen 7 und 8. Als Pufferlösungen sind alle üblichen Puffer geeignet; der Zusatz von Puffern ist bei Verwendung von Guanidinium-Hydrochlorid als Denaturierungsmittel wegen dessen Pufferwirkung nicht erforderlich. Vorzugsweise werden dem Fachmann bekannte Puffer, wie z.B. Tris oder Phosphat, verwendet. Überraschenderweise läßt sich das erfindungsgemäße Verfahren besonders vorteilhaft auch für Neurotrophine, auch unter sauren Bedingungen (pH 3 - 6,5), durchführen.

Das erfindungsgemäße Verfahren erfolgt unter Zusatz einer Disulfidkomponente. Bevorzugte Disulfidkomponenten sind z.B. GSSG, Cystamin und Cystin. Da die Derivatisierungsreaktion eine Gleichgewichtsreaktion zwischen Protein in der Thiolform und der Disulfidkomponente bzw. zwischen gemischtem Disulfid aus Protein und Disulfidkomponente einerseits und freien Thiolkomponenten, also verbliebenen Protein-Thiolgruppen sowie aus der Disulfidkomponente durch Reaktion mit dem Protein in Thiolform freigesetzter Thiol-Komponente, andererseits darstellt, muß die gewünschte Derivatisierungsreaktion durch einen hohen Überschuß der Disulfidkomponente erzwungen werden. Die hierfür erforderlichen Bedingungen sind von Protein zu Protein sehr unterschiedlich. Bevorzugt wird in einem Konzentrationsbereich der Disulfidkomponente von 10 mmol/l bis hin zur Sättigungsgrenze (bei GSSG z.B. abhängig vom pH-Wert des Ansatzes ca. 200 - 300 mmol/l, bei Cystamin ca. 700 mmol/l) gearbeitet, besonders bevorzugt ist der Konzentrationsbereich von 50 - 100 % der Sättigungskonzentration der Disulfidkomponente.

Weiter ist es bevorzugt, beim erfindungsgemäßen Verfahren ein Reduktionsmittel zuzusetzen. Besonders bevorzugt sind Reduktionsmittel aus der Mercaptangruppe, wie beispielsweise reduziertes Glutathion (GSH) oder 2-Mercaptoethanol, Dithioerythrit (DTE) oder Dithiothreit (DTT) in einer Konzentration von 0,01 - 50 mmol/l, vorzugsweise 0,1 - 10 mmol/l. Weiterhin bevorzugt sind Reduktionsmittel wie z.B. Salze der schwefligen Säure, z.B. Natriumsulfit. Der Zusatz eines dieser Reduktionsmittel ist für die erfolgreiche Durchführung der Reaktion zwar nicht Voraussetzung, abhängig vom behandelten Protein kann dieser Zusatz aber zu einer verbesserten Ausbeute bei der Reaktivierung des Proteins führen.

Das erfindungsgemäße Verfahren wird zweckmäßig bei Raumtemperatur, während einer Dauer von 0,1 - 100 Stunden, bevorzugt 1 - 24 Stunden, besonders bevorzugt 2 - 4 Stunden durchgeführt. Andere Bedingungen, wie z.B. Erwärmung bis auf etwa 60 °C oder Durchführung unter Kühlung bis auf etwa 0 °C, sind jedoch ebenfalls geeignet. Zur Verhinderung der Oxidation des Reduktionsmittels durch Luftsauerstoff und zum Schutz freier SH-Gruppen, ist es zweckmäßig, EDTA vorzugsweise in einer Menge von 1 - 100 mmol/l, besonders bevorzugt um ca. 10 mmol/l zuzusetzen. Ähnlich zweckmäßig zur Unterdrückung von radikalischen Nebenreaktionen, die beispielsweise in Thiol-haltigen Lösungen vor allem bei höheren pH-Werten ablaufen können, ist der Zusatz von Radikalfängern (Quenchern) wie z.B. Mannit, in einer Konzentration von 1 bis 1000 mmol/l, bevorzugt in einer Konzentration von 20 bis 200 mmol/l, besonders bevorzugt in einer Konzentration um 50 mmol/l bei der Naturierung und/oder Aufarbeiten der Proteine vorteilhaft.

Nach Solubilisierung/Derivatisierung wird vorzugsweise gegen eine Lösung, welche ein Denaturierungsmittel in einer denaturierenden Konzentration enthält, dialysiert, um die Disulfidkomponente und ggf. eingesetztes Reduktionsmittel zu entfernen. Zweckmäßig enthält die Lösung, gegen die dialysiert wird, das Denaturierungsmittel in der gleichen Konzentration wie die Denaturierungs-/Derivatisierungslösung. Ebenfalls bevorzugt ist es, gegen andere Denaturierungsmittel gleicher molarer Konzentration, gegen ca. 1 mmol/l HCl oder verdünnte Essigsäure, zu dialysieren. Es hat sich weiterhin als zweckmäßig erwiesen, die Disulfidkomponente nicht vollständig abzutrennen; wie bereits ausgeführt wurde, ist die Derivatisierungsreaktion eine Gleichgewichtsreaktion zwischen freien Thiol-Komponenten und (ggf. gemischten) Disulfid-Komponenten. Falls eine vollständige Derivatisierung aller Protein-Thiolgruppen nicht erfolgt, besteht nach Abtrennung der Disulfidkomponente die Gefahr, daß verbliebene freie Thiol-Komponente auf die vorhandenen gemischten Disulfide reduzierend einwirkt und so die Derivatisierungsausbeute bei der Lagerung des derivatisierten Proteins nachträglich zurückgehen kann. Angesichts des mit der Derivatisierung angestrebten Schutzes der Protein-Thiolgruppen gegen Oxidation und ähnliche destruktive Nebenreaktionen muß der Derivatisierungsgrad des behandelten Proteins deshalb möglichst hoch und stabil sein. Dies kann erreicht werden, indem entweder die Dialyse frühzeitig abgebrochen wird, bevor die Konzentration der Disulfidkomponente eine geeignete Konzentration unterschritten hat, oder indem bei der Dialyse gegen einen Dialysepuffer dialysiert wird, der die Disulfid-Komponente in eben der erforderlichen Konzentration enthält. Die erforderliche Konzentration, um den Derivatisierungsgrad während der Lagerung des derivatisierten Proteins aufrechtzuerhalten, ist vom jeweilig behandelten Protein und insbesondere vom Cystein-Gehalt des behandelten Proteins abhängig und kann in einem Konzentrationsbereich von 0 - 100 mmol/l liegen. Für die weitere Verwendung des derivatisierten Proteins zur Reaktivierungsreaktion muß beachtet werden, daß durch die Einbringung von Disulfidkomponente in den Reaktivierungsprozeß die hierbei angewandten Bedingungen für die gewollte oxidative Verknüpfung inter- oder intramolekularer Disulfidbrücken nicht oder zumindest nicht wesentlich beeinflußt werden. Aus diesem Grund hat sich eine Restkonzentration an Disulfidkomponente im derivatisierten Protein von von etwa 1 - 10 mmol/l als günstig erwiesen.

Ein weiterer Gegenstand der Erfindung ist ein Verfahren zur Herstellung eines naturierten Proteins aus seiner inaktiven schwerlöslichen Form, erhältlich nach rekombinanter Herstellung in Prokaryonten, welches dadurch gekennzeichnet ist, daß das Protein in seiner inaktiven schwerlöslichen Form mit einer Lösung eines Denaturierungsmittels in einer denaturierenden Konzentration und in Gegenwart einer Disulfidkomponente (molares Verhältnis Protein : Disulfidkomponente 1 : 1 bis 1 : 10000, vorzugsweise bis 1 : 1000) inkubiert, gelöst, derivatisiert und das gelöste Protein durch Änderung der stark denaturierenden Lösung in eine schwach oder nicht denaturierende Lösung eine biologisch aktive Konformation annimmt, wobei durch Zusatz eines Redoxsystems die Disulfidbindungen mit der Disulfidkomponente gelöst werden und im Protein intramolekular in der Weise neu gebildet werden, daß das Protein eine Konformation annimmt, in der es seine charakteristische biologische Aktivität besitzt.

Derartige schwach denaturierende Bedingungen können beispielsweise durch Verdünnung oder Dialyse, vorzugsweise in Gegenwart eines Reduktionsmittels, eingestellt werden. Schwach denaturierende Bedingungen sind, im Gegensatz zu stark denaturierenden Bedingungen, solche Bedingungen, bei denen das Protein in der Lage ist, seine aktive Konformation einzunehmen und in dieser Konformation stabil zu sein. In stark denaturierenden Bedingungen ist das Protein in dieser Form instabil und neigt zum Denaturieren, d. h. zum Verlust seiner stabilen dreidimensionalen Struktur und der energetisch günstigsten Disulfidbindung. Stark denaturierende Bedingungen sind beispielsweise gegeben in Lösungen von 4 - 9 mol/l Guanidinhydrochlorid. Schwach denaturierende Bedingungen sind beispielsweise zwischen 0,1 und 2 mol/l Guanidinhydrochlorid gegeben. Arginin wird bei der Naturierung zweckmäßig ebenfalls in Konzentrationen zwischen 0,1 und 1 mol/l eingesetzt

Unter Aktivität des Proteins ist eine biologische Aktivität des Proteins zu verstehen. Sofern es sich um ein natürlich vorkommendes oder ein Derivat eines natürlichen Proteins handelt, kann dessen biologische Aktivität über die immunologischen, zellbiologischen oder katalytischen Eigenschaften des Proteins bestimmt werden.

Bei der Aktivierung (Naturierung) ist es bevorzugt, bei einer GSH-Konzentration von 0,1 - 20 mmol/l, einer GSSG-Konzentration von 0,01 - 10 mmol/l ohne Denaturierungsmittel oder bei einer nichtdenaturierenden Konzentration eines Denaturierungsmittels zu arbeiten und die Reaktivierung über einen Zeitraum von 1 - 300 Stunden durchzuführen. Besonders bevorzugt beträgt die GSH-Konzentration hierbei 0,5 *-* 10 mmol/l und/oder die GSSG-Konzentration 0,1 - 10 mmol/l.

Das erfindungsgemäße Verfahren ist zur Naturierung einer Vielzahl von denaturierten Proteinen, insbesondere rekombinant hergestellten denaturierten Proteinen geeignet. Derartige Proteine sind beispielweise Proteasen, Wachstumsfaktoren, Proteinhormone, Cytokine, Plasminogenaktivatoren, Faktor Xa und insbesondere Neurotrophine. Neurotrophine sind Proteine, die sich insbesondere in Nervenzellen finden und die Differentation und das Überleben von Nervenzellen unterstützen. Neurotrophine (z. B. NGF, Brain derived nerve growth factor (BDNF), Neurotrophine 3, 4/5, 6) sind deshalb wertvolle Zelltherapeutica zur Behandlung von neurodegenerativen Erkrankungen, wie beispielsweise Polyneuropathien, Alzheimer Krankheit oder Verletzungen von Gehirn und Rückenmark.

Humaner Nerve growth factor (NGF) ist ein Protein, welches aus zwei Untereinheiten besteht (Homodimer). Die Fähigkeit, das Wachstum von sensorischen Neuronen und sympathischen Neuronen zu bewirken, wurde der β-Einheit zugeordnet. Reifer NGF besteht aus 118 Aminosäuren, enthält drei Disulfidbrücken und ist nicht glykosyliert. Biologisch aktiver NGF liegt als Dimer vor. Die DNA und Aminosäuresequenz von NGF wird in der EP-B 0 121 338 (USP 5,169,762) beschrieben. Aktives Protein konnte jedoch danach nicht erhalten werden. Die Herstellung von aktivem rekombinanten NGF ist beispielsweise in der EP-A 0 329 175 , EP-A 0 370 171, in Biochem. Biophys. Res. Commun. 171 (1990) 116 - 122, EP-A 0 414 151, Gene 70 (1988) 57 - 65, EP-A 0 450 386 und Gene 85 (1989), 109 - 114 beschrieben.

Brain derived neurotrophic factor (BDNF) wurde von Leibrock et al., Nature 341 (1989) 149 - 152 beschrieben. BDNF unterstützt das Überleben von sensorischen Neuronen im zentralen Nervensystem und scheint Erfolge bei der Behandlung der Parkinson'schen Krankheit zu haben. Rekombinanter BDNF kann beispielsweise nach WO 91/03568 in CHO-Zellen und nach WO 92/22665 in Prokaryonten hergestellt werden.

Die folgenden Beispiele, Publikationen und das Sequenzprotokoll erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beispiel 1

### Expression von NGF in E.coli

### a) Expressionsplasmid

Auf Basis der von Ullrich et al. (Nature 303: 821, 1983) publizierten Sequenz und unter Einbeziehung einiger Abänderungen, insbesondere des 5' gelegenen Anteils, wurde das NGF-Gen, das für den maturen Teil kodiert, synthetisch hergestellt (SEQ ID NO:4). Dazu wurde die Methode von Beattie and Fowle (1991, Nature 352: 548 - 549) herangezogen. Um die Klonierung zu erleichtern, wurde an das 5'-Ende eine Schnittstelle für das Restriktionsenzym EcoRI und an das 3'-Ende eine Schnittstelle für das Restriktionsenzym HindIII eingefügt Die synthetisierte Nukleinsäure wurde mit den Enzymen EcoRI und HindIII restringiert und mit dem Expressionsvektor pA27fd (beschrieben in EP-A 0 382 174), der zuvor mit EcoRI und partiell mit HindIII verdaut worden war, ligiert. Der Ligationszusatz wurde zusammen mit dem Helferplasmid pUBS520 in E.coli transformiert (Brinkmann et al., Gene 85 (1989), 109-114).

Die Selektion der Klone erfolgte über die durch die Plasmide vermittelte Ampicillin- bzw. Kanamycin-Resistenz. Das erhaltene Plasmid pNGF23fd enthält, im Vergleich zum Ausgangsplasmid pA27fd ein kleineres EcoRI/HindIII-Fragment mit einer Größe von etwa 400 bp.

### b) Expression in E.coli

Zur Überprüfung der Expressionsleistung wurde der mit den Plasmiden pNGF23fd und pUBS520 transformierte E.coli-Stamm in LB-Medium (Sambrook et al., 1989, Molecular Cloning, Cold Spring Harbor) in Gegenwart von Ampicillin und Kanamycin (jeweils 50 µg/ml Endkonzentration) bis zu einer OD von 550 nm angezüchtet. Die Expression wurde durch Zugabe von 5 mM IPTG initiiert. Die Kultur wurde für weitere 4 Stunden inkubiert. Im Anschluß daran wurden die E.coli durch Zentrifugation gesammelt und in Puffer resuspendiert (50 mM Tris-HCl pH 8, 50 mM EDTA); durch Beschallung wurde die Lyse der E.coli herbeigeführt. Durch erneute Zentrifugation wurden die unlöslichen Proteinfraktionen gesammelt und in oben genanntem Puffer durch Beschallung resuspendiert. Die Suspension wurde mit 1/4 Volumen Auftragspuffer (250 mM Tris-HCl pH 6,8, 0.01 mEDTA, 5 % SDS, 5 % Mercaptoethanol, 50 % Glycerin und 0.005 % Bromphenolblau) versetzt und mit Hilfe eines 12,5 % SDS-Polyacrylamidgels analysiert. Als Kontrolle wurde die gleiche Präparation mit einer Kultur von E.coli (pNGF23fd, pUBS520), die nicht mit IPTG versetzt worden war, durchgeführt und im Polyacrylamidgel aufgetragen. In der Präparation der IPTG-induzierten Kultur kann man, nach Anfärben des Gels mit 0.2 % Coomassie-Blue R250 (gelöst in 30 % Methanol und 10 % Essigsäure), eine deutliche Bande mit einem Molekulargewicht (Abgleich gegenüber Standardproteingemisch von Biorad "H+L") von etwa 14 kD erkennen. Diese Bande ist in der Präparation der nicht-induzierten E.coli Zellen nicht vorzufinden.

### Beispiel 2

### Präparation von Inclusion Bodies (= IBs)

Zur Präparation der rec. NGF-enthaltenden IBs wurde der in Beispiel 1 beschriebene Expressionsstamm E.coli in einem 10 1-Fermenter für 8 Stunden fermentiert. Die Induktion der Expression von NGF erfolgte durch die Zugabe von IPTG in der logarithmischen Wachstumsphase ca. 4 Stunden nach Fermentationsbeginn.

690 g Biomasse wurden nach 8 Stunden Fermentation mittels Zentrifugation geerntet. Die Biomasse wurde in 3,5 1 0,1 mol/l Tris-HCl pH 7 suspendiert und nach Zugabe von 0,7 g Lysozym, 7 mg DNase und 0,4 mmol/l MgSO₄ 20 Minuten bei 0°C inkubiert. Der vollständige Zellaufschluß wurde anschließend mittels Hochdruckdispersion mit 1000 bar durchgeführt. Zur Aufschlußlösung wurde nochmals DNase ad 0,1 mg/ml und MgSO₄ ad 2 mmol/l zugegeben und die Lösung 30 Minuten bei 20°C inkubiert. Nach der DNase-Behandlung wurde die Lösung mit dem halben Volumen 6 % Brij 35, 1,5 mol/l NaCl, 60 mmol/l EDTA, pH 7.0 verdünnt und 20 Minuten im Eisbad inkubiert. Unlösliche Bestandteile (IBs) wurden anschließend durch Zentrifugation abgetrennt. Der Niederschlag wurde im 3fachen Volumen 0,1 mol/l Tris-HCl, 20 mmol/l EDTA, pH 6.5 (TE-Puffer) suspendiert. Nach 30 Minuten Inkubation bei 20°C wurden die IBs durch erneute Zentrifugation geerntet. Die folgende Resuspension des Niederschlags erfolgte im 3fachen Volumen TE-Puffer. Nach 30 Minuten Inkubation bei 20°C wurden die IBs durch eine weitere Zentrifugation im Niederschlag erhalten.

Zur Bestimmung des Anteils an rh-NGF in den IBs wurden 500 mg IBs (Naßgewicht) mit einer Lösung aus 7,5 mol/l Guanidinium-HCl (GdmHCl) und 10 mmol/l EDTA, pH 6.0 auf 10 ml aufgefüllt und 2 Stunden suspendiert. Der Proteingehalt der Lösung wurde mittels Proteinbestimmung nach Biuret (Boehringer Mannheim, Bestell-Nr. 124281) bestimmt. Der Anteil von rh-NGF am Gesamtprotein in den gelösten IBs wurde nach Auftrennung der mit SDS denaturierten und mit DTE reduzierten Proteine mittels SDS-Kapillarelektrophorese durch Vergleich der Peakfläche mit der von Standard-NGF (z.B. Boehringer Mannheim, Bestell-Nr. 1457616) oder nach Auftrennung der Proteine mittels SDS-Gelelektrophorese durch densitometrische Vermessung der Probenbahnen ermittelt. Die aus 10 1 Fermentationsbrühe isolierten IBs enthielten ca. 6 g rh-NGF.

### Beispiel 3

### Solubilisierung und Derivatisierung von rh-NGF

### a) Herstellung von Solubilisat

IBs wurden in einer Lösung aus 7,5 mol/l GdmHCl, 0,1 mol/l Tris-HCl, 10 mmol/l EDTA und 0,1 mol/l DTT, pH 8.5 zu einer Konzentration von 20 bis 200 g IBs/l suspendiert und 2 Stunden bei 20 - 25°C gerührt. Anschließend wurde die Lösung mit 25%iger HCl auf pH 3 eingestellt und auf ca. 4°C gekühlt. Das so erhaltene Solubilisat wurde bei ca. 4°C in einer Crossflow-Filtrationsanlage über eine Ultranitrationsmembran mit Ausschlußgrenze 10 kDa gegen 6 - 10 Volumina 7,5 mol/l GdmHCl, 10 mmol/l EDTA, pH 3 diafiltriert oder im Dialyseschlauch mehrfach gegen den gleichen Puffer dialysiert.

### b) Herstellung von Derivat aus Solubilisat (Stand der Technik)

Das aus Beispiel 3a erhaltene DTT-frei dialysierte Solubilisat wurde mit 20 mmol/l GSSG versetzt und durch Titration mit einer Lösung aus 1 mol/l Tris auf pH 7.5 eingestellt. Der erhaltene Ansatz wurde 2 Stunden bei ca. 20 - 25°C inkubiert und dann mit 25%iger HCl auf pH 6 eingestellt und auf ca. 4°C abgekühlt. Das so erhaltene Derivat wurde bei ca. 4°C in einer Crossflow-Filtrationsanlage über eine Ultrafiltrationsmembran mit Ausschlußgrenze 10 kDa gegen 6 - 10 Volumina 7,5 M GdmHCl, 10 mmol/l EDTA, pH 6 diafiltriert oder im Dialyseschlauch mehrfach gegen den gleichen Puffer dialysiert.

### c) Herstellung von Derivat direkt aus den IBs (Erfindungsgemäßes Verfahren)

IBs wurden in einer Lösung aus 7,5 mol/l GdmHCl, 0,1 mol/l Tris, 10 - 200 mmol/l GSSG, 10 mmol/l EDTA, pH 6 zu einer Konzentration von 20 bis 200 g IBs/l suspendiert und 3 Stunden bei 20 - 25°C gerührt. Das so erhaltene Derivat wurde bei ca. 4°C in einer Crossflow-Filtrationsanlage über eine Ultrafiltrationsmembran mit Ausschlußgrenze 10 kDa gegen 6 - 10 Volumina 7,5 mol/l GdmHCl, 10 mmol/l EDTA, pH 6 diafiltriert oder im Dialyseschlauch mehrfach gegen den gleichen Puffer dialysiert.

Die direkte Derivatisierung aus IBs wurde in analoger Weise bei pH-Werten von 3 - 10 bei der Derivatisierung und pH 6 bei der Diafiltration durchgeführt, der pH-Wert wurde vor der Dialyse mit NaOH bzw. HCl auf 6 eingestellt. Bei pH-Werten unter 6 wurde die GSSG-Konzentration bis 300 mmol/l erhöht. Ggf. ungelöstes GSSG wurde vor Beginn der Derivatisierung durch Zentrifugation entfernt.

Der Nachweis der erfolgten Derivatisierung wurde mittels SDS-Gelelektrophorese und Massenspektroskopie (MALDI-MS) geführt. Hierbei wurde gezeigt, daß der Derivatisierungsgrad nach Beispiel 3c unabhängig vom pH-Wert bei der Derivatisierung deutlich höher lag als bei der nach Stand der Technik durchgeführten Derivatisierung (Beispiel 3b).

Das Naturierungsverhalten von Derivat und Solubilisat wurde mit frischem und bei 4°C gelagertem Material untersucht (Details s. Beispiel 4). Während die nach 3c hergestellten Derivate unabhängig vom pH-Wert der Herstellung nach 4 Wochen ein unverändertes Naturierungsverhalten zeigten (Ausbeute ca. 100 % des Startwertes), sank die Naturierungsausbeute beim Solubilisat (3a) auf ca. 60 % und beim nach Stand der Technik hergestellten Derivat (3b) auf ca. 80 % ab. Dies korrespondiert mit den MALDI-MS-Daten, d.h. die Stabilität des Derivats ist erwartungsgemäß abhängig vom Derivatisierungsgrad.

### Beispiel 4

### Naturierung von rh-NGF

Zur Darstellung von biologisch aktivem rh-NGF aus den in Beispiel 3 hergestellten Solubilisaten/Derivaten wurden die rh-NGF in inaktiver, löslicher Form enthaltenden Lösungen 20- bis 500-fach bei ca. 4°C in Naturierungspuffer, bestehend aus 1 mol/l Tris-HCl, 0,5 mol/l Arginin, 1 mmol/l EDTA, 1 mmol/l GSH, pH 9.1 verdünnt.

Zum Nachweis von naturiertem rh-NGF wurden die Ansätze nach einer Inkubationszeit von 24 h mittels Reversed-Phase-Chromatographie an einer POROS R1/H-Säule (2,1 x 100 mm, Perseptive Biosystems, Freiburg, Germany) quantifiziert. Als Startpuffer diente 5% Acetonitril in H₂O (0,1% TFA), die Elution erfolgte mit einem Gradienten bis 80% Acetonitril in H₂O (0,1% TFA) über 20 min bei einer Flußrate von 1 ml/min. Nativer NGF wurde durch Bewertung der Eluat-Fraktionen im Bioassay (s.u.) identifiziert.

Zur Bestimmung der Konzentrationen an naturiertem, biologisch aktiven rh-NGF in den HPLC-Fraktionen oder direkt in Naturierungslösungen wurde die Stimulierbarkeit durch rh-NGF von sensorischen Neuronen aus dissoziierten Dorsalwurzelganglien von Hühnerembryonen (Embryonaltag 8) zum Austreiben von Dendriten (= DRG-Test = dorsal root ganglion assay, Levi-Montalcini, R., Meyer, H. und Hamburger, V. 1954, Cancer Res. 14, 49-57; Varon, S., Nomura, J., Perez-Polo, J.R. und Shooter, E.M., 1972, Meth. in Neurochemistry 3, 203-229; EP-A 0335673, S14-15, Beispiel C) verwendet.

Die HPLC-Fraktionen wurden in einer Verdünnungsreihe in Konzentrationen von c=100 ng/ml bis c=100 pg/ml in 1:2 Verdünnungsschritten in 48-Well-Platten getestet. Dabei wurden in Zellkulturplatten der Fa. Falcon 300 µl Medium (F14-Medium; Coon, M.G. und Weiß, M.G., 1969, Proc. Natl. Acad. Sci. USA 62, 852-859) und 100 µl der Zellsuspension plus 100 µl der oben angegebenen Verdünnungen (=Endkonzentrationen c=20 ng/ml bis c=20 pg/ml) gemischt und 48 Stunden bei 37°C und 3.5% CO₂ inkubiert. Als Maß für die biologische Aktivität wurde die Anzahl der Zellen mit ausgebildeten Dendriten quantifiziert. Als Referenz wurde eine Lösung bekannter Konzentration von 2.5 S NGF aus Subcnaxillaris-Drüsen der Maus (Fa. Boehringer Mannheim) verwendet. Naturierungsansätze wurden nach Zentrifugation und ggf. Vorverdünnung mit F14-Medium analog untersucht.

### Beispiel 5

### Klonierung der katalytischen Domäne des FX Proteasegens

### (Plasmid: pFX-CD)

### Methoden

### Rekombinante DNA-Technik

Zur Manipulation von DNA wurden Standardmethoden benutzt, wie sie bei Sambrook, J. et al. (1989) In: Molecular cloning: A laboratory manual. Cold Spring Harbor Laboratory Press, Cold Spring Harbor, New York, beschrieben sind. Die verwendeten molekularbiologischen Reagenzien wurden nach den Angaben des Herstellers eingesetzt.

### Proteinbestimmung

Die Proteinkonzentration der Proteasevariante rFX-EGF2-AP-CD wurde durch Bestimmung der optischen Dichte (OD) bei 280 nm unter Verwendung des anhand der Aminosäuresequenz errechneten molaren Extinktionskoeffizienten (ε = 43490 cm²/ mol) ermittelt.

### Expressionsvektor

Der Vektor zur Expression der Blutgerinnungsproteasevarianten basiert auf dem Expressionsvektor pSAM-CORE für core-Streptavidin. Die Herstellung und Beschreibung des Plasmids pSAM-CORE ist in der WO 93/09144 von Kopetzki, E. et al. beschrieben.

Das core-Streptavidin Gen wurde durch das gewünschte Proteasevariantengen im pSAM-CORE Vektor ersetzt.

### Klonierung

Die FX cDNA von Bp-Position 649-1362, kodierend für die FX Proteasedomäne von Aminosäureposition 217-454 (cDNA Sequenz- und Aminosäuresequenz-Numerierung entsprechend der Publikation von Kaul, R.K. et al. (Gene 41 (1986) 311-314; wurde in einer "Polymerase Chain Reaktion" (PCR) gemäß der Methode von Mullis, K.B. und Faloona, F.A. (Methods Enzymol. 155, (1987) 355-350) unter Verwendung der PCR Primer N1 (SEQ ID NO: 1) und N2 (SEQ ID NO: 2) und einer kommerziell erhältlichen humanen Leber cDNA Genbank (Vektor: Lamba ZAP® II) der Firma Stratagene (La Jolla, CA, U.S.A.) als Template DNA, amplifiziert. Mittels der PCR Primer wurde am 5'-Ende der kodierenden Region eine singuläre BspHI Schnittstelle und ein ATG-Startkodon und am 3'-Ende der kodierenden Region eine singuläre HindIII Schnittstelle eingeführt.

Das ca. 740 Bp lange PCR Produkt wurde mit den Restriktionsendonukleasen BspHI und HindIII verdaut und das ca. 725 Bp lange BspHI/HindIII-FX Fragment nach Reinigung durch Agarosegelelektrophorese in das ca. 2,55 kBp lange NcoI/HindIII-pSAM-CORE Vektorfragment ligiert. Das gewünschte Plasmid pFX-CD wurde durch Restriktionskartierung identifiziert und die durch PCR isolierte FX cDNA Sequenz durch DNA Sequenzierung überprüft.

### Beispiel 6

### Klonierung des FX Proteasegens mit EGF2-Domäne, Aktivierungspeptid und katalytischer Domäne (Plasmid: pFX-EGF2-AP-CD)

Die FX cDNA von Bp-Position 322-1362, kodierend für die EGF2-Domäne, das Aktivierungspeptid und die katalytische Proteasedomäne von Aminosäureposition 108-454 wurde mittels PCR unter Verwendung der PCR Primer N3 (SEQ ID NO: 3) und N2 (SEQ ID NO: 2) und einer kommerziell erhältlichen humanen Leber cDNA Genbank (Vektor: Lamba ZAP® II) der Firma Stratagene (La Jolla, CA, U.S.A.) als Template DNA amplifiziert. Mittels der PCR Primer wurde am 5'-Ende der kodierenden Region ein ATG-Startkodon und eine singuläre EcoRI Schnittstelle und am 3'-Ende der kodierenden Region eine singuläre HindIII Schnittstelle eingeführt.

Das ca. 1,09 KBp lange PCR Produkt wurde mit den Restriktionsendonukleasen EcoRI und BstEII verdaut und das ca. 1,02 KBp lange EcoRI/BstEII-FX Fragment nach Reinigung durch Agarosegelelektrophorese in das ca. 2,58 KBp lange EcoRI/BstEII-pFX-CD Vektorfragment (Beispiel 5) ligiert. Das gewünschte Plasmid pFX-EGF2-AP-CD wurde durch Restriktionskartierung identifiziert und die durch PCR isolierte FX cDNA Sequenz durch DNA Sequenzierung überprüft.

### Beispiel 7

### a) Expression des Proteasegens in E. coli

Zur Expression des FX-EGF2-AP-CD Proteasegens wurde ein E. coli K12 Stamm (z.B. UT5600, Grodberg, J. und Dunn, J.J., J. Bacteriol. 170 (1988) 1245-1253) mit dem im Beispiel 6 beschriebenen Expressionsplasmid pFX-EGF2-AP-CD (Ampicillin-Resistenz) und dem lacI^{q}-Repressorplasmid pUBS520 (Kanamycin-Resistenz, Herstellung und Beschreibung siehe: Brinkmann, U. et al., Gene 85 (1989) 109-114) transformiert.

Die transformierten UT5600/pUBS520/pFX-EGF2-AP-CD Zellen wurden in Schüttelkultur in DYT-Medium (1% (w/v) Hefeextrakt, 1% (w/v) Bacto Tryptone, Difco, und 0,5% NaCl) mit 50-100 mg/l Ampicillin und 50 mg/l Kanamycin bei 37°C bis zu einer optischen Dichte bei 550 nm (OD₅₅₀) von 0,6-0,9 angezogen und anschließend mit IPTG (1=5 mmol/l Endkonzentration) induziert. Nach einer Induktionsphase von 4 - 8 Stunden (Std.) bei 37°C wurden die Zellen durch Zentrifugation (Sorvall RC-5B Zentrifuge, GS3 Rotor, 6000 UPM, 15 min) geerntet, mit 50 mmol/l Tris-HCl Puffer, pH 7,2 gewaschen und bis zur Weiterverarbeitung bei -20°C gelagert. Die Zellausbeute aus einer 11 Schüttelkultur betrug 4-5 g (Naßgewicht).

### b) Expressionsanalyse

Die Zellpellets aus jeweils 1 ml abzentrifugiertem Anzuchtmedium (UT5600/pUBS520/pFX-EGF2-AP-CD Zellen) wurden in 0,25 ml 10 mmol/l Tris-HCl, pH 7,2 resuspendiert und die Zellen durch Ultraschallbehandlung (2 Pulse a 30 s mit 50% Intensität) mit einem Sonifier® Cell Disruptor B 15 der Firma Branson (Heusenstamm, BRD) aufgeschlossen. Die unlöslichen Zellbestandteile wurden sedimentiert (Eppendorf 5415 Zentrifuge, 14000 UPM, 5 min) und der Überstand mit 1/5 Volumen (Vol) 5xSDS-Probenpuffer (1xSDS-Probenpuffer: 50 mmol/l Tris-HCl, pH 6,8, 1% SDS, 1% Mercaptoethanol, 10% Glycerin, 0.001% Bromphenolblau) versetzt. Die unlösliche Zelltrümmerfraktion (Pellet) wurde in 0,3 ml 1xSDS-Probenpuffer mit 6 - 8 mol/l Harnstoff resuspendiert, die Proben 5 min bei 95°C inkubiert und erneut zentrifugiert. Danach wurden die Proteine durch SDS-Polyacrylamid Gelelektrophorese (PAGE) aufgetrennt (Laemmli, U.K., Nature 227 (1970) 680-685) und mit Coomassie Brilliant Blue R Farbstoff angefärbt.

Die in E. coli synthetisierte FX-EGF2-AP-CD Proteasevariante war homogen und wurde ausschließlich in der unlöslichen Zelltrümmerfraktion gefunden ("inclusion bodies", IBs). Die Expressionshöhe betrug ca. 50% bezogen auf das E. coli Gesamtprotein.

### Beispiel 8

### Zellyse, Solubilisierung und Präparation der "inclusion bodies" (IBs)

Das Zellpellet aus 3 1 Schüttelkultur (ca. 15 g Naßgewicht) wurde in 75 ml 50 mmol/l Tris-HCl, pH 7,2, resuspendiert. Die Suspension wurde mit 0,25 mg/ml Lysozym versetzt und 30 min bei 0°C inkubiert. Nach Zugabe von 2 mmol/l MgCl₂ und 10 µg/ml DNase I (Boehringer Mannheim GmbH, Kat.-Nr. 104159) wurden die Zellen mechanisch mittels Hochdruckdispersion in einer French® Press der Firma SLM Amico (Urbana, IL, U.S.A.) aufgeschlossen. Anschließend wurde die DNA 30 min bei Raumtemperatur (RT) verdaut. Der Ansatz wurde mit 37,5 ml 50 mmol/l Tris-HCl pH 7,2, 60 mmol/l EDTA, 1,5 mol/l NaCl, 6% Brij X-100 versetzt, weitere 30 min bei RT inkubiert und in einer Sorvall RC-5B Zentrifuge (GSA Rotor, 12000 UPM, 15 min) zentrifugiert. Der Überstand wurde verworfen, das Pellet mit 100 ml 50 mmol/l Tris-HCl, pH 7,2, 20 mmol/l EDTA versetzt, 30 min unter Rühren bei 4°C inkubiert und erneut sedimentiert. Der letzte Waschschritt wurde wiederholt. Die gereinigten IBs (1,5-2,0 g Naßgewicht, 25-30% Trockenmasse, 100-150 mg Protease) wurden bis zur Weiterverarbeitung bei -20°C gelagert.

### Beispiel 9

### Solubilisierung und Reduktion / Derivatisierung und Dialyse der IBs

Die gereinigten IBs wurden in einer Konzentration von 100 mg IB-Pellet (Naßgewicht)/ml entsprechend 5 - 10 mg/ml Protein in 6 mol/l Guanidinium-HCl, 100 mmol/l Tris-HCl, 20 mmol/l EDTA, pH 8,0 suspendiert und Aliquots in Gegenwart von
200 mmol/l GSSG oder 200 mmol/l GSH
unter Rühren bei RT in 1-3 Std. gelöst. Anschließend wurde der pH auf pH 5,0 eingestellt und die unlöslichen Bestandteile durch Zentrifugation (Sorvall RC-5B Zentrifuge, SS34 Rotor, 16000 UPM, 15 min) abgetrennt und gegen 6 mol/l Guanidinium-HCl, pH 5,0 bei 4°C für 24 Std. dialysiert. Die Derivatisierung wurde mittels SDS-PAGE nachgewiesen.

### Beispiel 10

### Naturierung von FX-EGF2-AP-CD in Abhängigkeit von der Reduktion / Derivatisierung

Die Naturierung der in 6 mol/l Guanidinium-HCl solubiliserten und mit 100 mmol/l DTE reduzierten, oder mit unterschiedlichen Konzentrationen von GSSG/GSH derivatisierten FX-EGF2-AP-CD Proteasevariante wurde bei 4°C durch einmalige Zugabe von jeweils 50µl IB-Solubilisat/Derivat zu 5 ml des Naturierungspuffers (50 mmol/l Tris-HCl / 0,6 mol/l Arginin /10 mmol/l CaCl₂ / 2 mmol/l EDTA / 2 mmol/l GSH / 0,5 mmol/l GSSG, pH 8,5) bewirkt.

Das naturierte Protein wurde zweimal gegen je 100 Vol 50 mmol/l Tris-HCl, 150 mmol/l NaCl, 5 mmol/l CaCl₂, 0,1% Polyethylenglycol 8000 (PEG 8000), pH 8,0 für 8-16 Std. bei 4 °C dialysiert. Präzipitiertes Protein wurde durch Zentrifugation abgetrennt (Eppendorf 5415 Zentrifuge, 14000 UPM, 5 min) und der klare Überstand für die Aktivierung eingesetzt.

### Beispiel 11

### Aktivierung der rFX-EGF2-AP-CD Proteasevariante mit RVV-X

Jeweils 1 ml der naturierten und dialysiertem rFX-EGF2-AP-CD Proben wurden mit 10 µl einer "Russels viper venom" (RVV) Lösung (1 mg/ml Lyophilisat gelöst in 20 mmol/l Tris-HCl, pH 7,6) der Firma Sigma Aldrich Chemie GmbH (Deisenhofen, BRD) versetzt und bei 37°C für 1-2 Tage inkubiert. Der zeitliche Verlauf der enzymatischen rFX-EGF2-AP-CD Aktivierung wurde mit dem chromogenen Peptidsubstrat Chromozym X (siehe Beispiel 12) bis zur Vollständigkeit des Verdaus (Plateau, maximale Aktivierung) verfolgt. Dazu wurden aus dem Reaktionsansatz im Abstand von 4-6 Std. Proben (20 µl) entnommen und die generierte FXa Aktivität bestimmt.

### Beispiel 12

### FXa Aktivitätstest

Die Aktivität von naturierten und aktivierten rFXa-EGF2-AP-CD wurde mit dem chromogenen Substrat Chromozym X der Firma Boehringer Mannheim GmbH (Mannheim, BRD, Kat.-Nr. 789763) bestimmt. 20 µl Probe wurden mit 180 µl 50 mmol/l Tris-HCl, 150 mmol/l NaCl, 5 mmol/l CaCl₂, 0,1% PEG 8000, pH 8,0 und 20 µl 4 mmol/l Chromozym X in einer Mikrotiterplatte bei RT versetzt und die lineare Anfangsgeschwindigkeit (ΔE/min) durch Extinktionsmessungen bei einer Wellenlänge von 405 nm in einem ELISA-Reader bestimmt.

### Testprinzip:

### Beispiel 13

### Bestimmung der Naturierungseffizienz

Zur Berechnung der Naturierungseffizienz wurde die generierte rFXa-EGF2-AP-CD Aktivität (Plateauwert) verwendet. Der höchste Wert innerhalb der Meßreihe diente als Bezugsgröße, die auf 100% gesetzt wurde.

Das reduzierte Protein lieferte eine Naturierungsausbeute von ca. 60 % im Vergleich zum derivatisierten Protein.

### Referenzliste

Bailey, J.L., Cole, R.D., J. Biol. Chem. 234 (1959) 1733-1739.
Beattie and Fowle, Nature 352 (1991) 548 - 549
Biochem. Biophys. Res. Commun. 171 (1990) 116 - 122
Brinkmann et al., Gene 85 (1989) 109 -114
Cole, R.D., Meth. Enzymol. 11 (1967) 206-208.
Coon, M.G. und Weiß, M.G., Proc. Natl. Acad. Sci. USA 62 (1969), 852-859
EP-A 0 093 619
EP-A 0 114 506
EP-A 0 114 507
EP-A 0 241022
EP-A 0 253 823
EP-A 0 329 175
EP-A 0 335 673
EP-A 0 361 475
EP-A 0 364 926
EP-A 0 370 171
EP-A 0 382 174
EP-A 0 414 151
EP-A 0 450 386
EP-B 0 121 338 (USP 5,169,762)
Gene 70 (1988) 57 - 65
Grodberg, J.; Dunn, J.J.: J. Bacteriol. 170 (1988) 1245-1253
Heath, W.F. et al., J. Biol. Chem. 267 (1992) 419 - 425
Kaul, R.K.; Hildebrand, B.; Roberts, S.; Jagadeeswaran, P., Gene 41 (1986) 311-314
Laemmli, U.K., Nature 227 (1970) 680-685 .
Leibrock et al., Nature 341 (1989) 149 - 152Levi-Montalcini, R., Meyer, H. und Hamburger, V., Cancer Res. 14 (1954) 49-57
Marston F.A.O., Biochem. J. 214 (1986) 1 - 12
Mullis, K.B.; Faloona, F.A, Methods Enzymol. 155 (1987) 350-355
Sambrook, J.; Fritsch, E.F.; Maniatis,T.: Molecular cloning: A laboratory manual. Cold Spring Harbor Press, Cold Spring Harbor, New York, (1989).
Thannhauser, T.W., Konishi, Y., Scheraga, H.A., Analyt. Biochem 138 (1984) 181-188.
Thannhauser, T.W., Scheraga, H.A., Biochemistry 24 (1985) 7681-7688.
Ullrich et al., Nature 303 (1983) 821 - 825
US-Patent 4,933,434
Varon, S., Nomura, J., Perez-Polo, J.R. und Shooter, E.M., Meth. in Neurochemistry 3 (1972) 203-229
Wetzel, R et al., Gene 16 (1981) 63 - 71
WO 87/02673
WO 91/03568
WO 91/08762
WO 92/22665
WO 93/09144
WO 93/19084
WO 95/30686

## Patentansprüche

1. Verfahren zur Herstellung von gemischten Disulfiden aus einem Protein und einer Disulfidkomponente, **dadurch gekennzeichnet, daß** das Protein in einer inaktiven schwerlöslichen Form mit einer Lösung eines Denaturierungsmittels in einer denaturierenden Konzentration und in Gegenwart einer Disulfidkomponente inkubiert, gelöst und derivatisiert wird.

2. Verfahren nach Anspruch 1, **dadurch gekennzeichnet, daß** nach Derivatisierung die Disulfidkomponente entfernt wird.

3. Verfahren nach den Ansprüchen 1 oder 2, **dadurch gekennzeichnet, daß** die Derivatisierung unter sauren Bedingungen erfolgt.

4. Verfahren nach den Ansprüchen 1 - 3, **dadurch gekennzeichnet, daß** freie SH-Gruppen am Protein durch Zusatz von EDTA geschützt werden.

5. Verfahren nach den Ansprüchen 1 - 4, **dadurch gekennzeichnet, daß** als Disulfidkomponente GSSG, Cystamin oder Cystin verwendet wird.

6. Verfahren nach den Ansprüchen 1 - 5, **dadurch gekennzeichnet, daß** die Disulfidkomponente in einer Konzentration von mindestens 1 mmol/l verwendet wird.

7. Verfahren zur Herstellung eines biologisch aktiven Proteins aus seiner inaktiven schwerlöslichen Form, erhältlich nach rekombinanter Herstellung in Prokaryonten, **dadurch gekennzeichnet, daß** das Protein in seiner inaktiven schwerlöslichen Form mit einer Lösung eines Denaturierungsmittels in einer denaturierenden Konzentration und in Gegenwart einer Disulfidkomponente gelöst, das gelöste Protein durch Änderung der stark denaturierenden Lösung in eine nicht oder schwach denaturierende Lösung eine biologisch aktive Konformation annimmt, wobei die Disulfidbindungen mit der Disulfidkomponente gelöst werden und im Protein intramolekular in der Weise neu gebildet werden, daß das Protein eine Konformation einnimmt, die es zu einer biologischen Aktivität befähigt.

8. Verfahren nach Anspruch 7, **dadurch gekennzeichnet, daß** das naturierte Protein eine Protease, ein Wachstumsfaktor, ein Proteinhormon, ein Neurotrophin oder ein Cytokin ist.

## Claims

1. Method for preparing mixed disulfides from a protein and a disulfide component, **characterized in that** the protein is incubated in an inactive sparingly soluble form with a solution of a denaturing agent at a denaturing concentration and in the presence of a disulfide component, dissolved and derivatized.

2. Method as claimed in claim 1, **characterized in that** the disulfide component is removed after derivatization.

3. Method as claimed in claims 1 or 2, **characterized in that** the derivatization is carried out under acidic conditions.

4. Method as claimed in claims 1 - 3, **characterized in that** free SH groups on the protein are protected by adding EDTA.

5. Method as claimed in claims 1 - 4, **characterized in that** GSSG, cystamine or cystine is used as the disulfide component.

6. Method as claimed in claims 1 - 5, **characterized in that** the disulfide component is used at a concentration of at least 1 mmol/l.

7. Method for preparing a biologically active protein from its inactive sparingly soluble form obtainable after recombinant production in prokaryotes, **characterized in that** the protein in its inactive sparingly soluble form is dissolved with a solution of a denaturing agent at a denaturing concentration and in the presence of a disulfide component, the dissolved protein adopts a biologically active conformation by changing the strongly denaturing solution into a non-denaturing or weakly-denaturing solution in the process of which the disulfide bonds with the disulfide component are broken and new intramolecular disulfide bonds are formed in the protein in such a manner that the protein adopts a conformation enabling it to have a biological activity.

8. Method as claimed in claim 7, **characterized in that** the renatured protein is a protease, a growth factor, a protein hormone, a neurotrophin or a cytokine.

## Revendications

1. Procédé de production de disulfures mélangés issus d'une protéine, et d'un composant disulfure, **caractérisé en ce que** la protéine est incubée, dissoute et dérivée dans une forme inactive peu soluble avec une solution d'un agent dénaturant en une concentration dénaturante et en présence d'un composant disulfure.

2. Procédé selon la revendication 1, **caractérisé en ce que** le composant disulfure est retiré après dérivation.

3. Procédé selon la revendication 1 ou 2, **caractérisé en ce que** la dérivation s'effectue dans des conditions acides.

4. Procédé selon les revendications 1 à 3, **caractérisé en ce que** dés groupes SH libres sur la protéine sont protégés par l'ajout d'EDTA.

5. Procédé selon les revendications 1 à 4, **caractérisé en ce que** l'on utilise comme composant disulfure, le GSSG, la cystamine ou la cystine.

6. Procédé selon les revendications 1 à 5, **caractérisé en ce que** le composant disulfure est utilisé en une concentration d'au moins 1 mmol/l.

7. Procédé de production d'une protéine biologiquement active à partir d'une forme inactive peu soluble, obtenue par production recombinante dans des cellules procaryotes, **caractérisé en ce que** la protéine est dissoute sous sa forme inactive peu soluble avec une solution d'agent dénaturant en une concentration dénaturante et en présence d'un composant disulfure, **en ce que** la protéine dissoute adopte par modification de la solution fortement dénaturée dans une solution non dénaturante ou faiblement dénaturante, une conformation biologiquement active, les liaisons disulfure avec le composant disulfure étant défaites et étant nouvellement formées sur le plan intramoléculaire dans la protéine, de telle sorte que la protéine adopte une conformation qui permette une activité biologique.

8. Procédé selon la revendication 7, **caractérisé en ce que** la protéine dénaturée est une protéase, un facteur de croissance, une hormone protéique, une neurotrophine ou une cytokine.


### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Germany
      (F) POSTLEITZAHL: D-68305
      (G) TELEFON: 08856/60-3446
      (H) TELEFAX: 08856/60-3451
   (ii) BEZEICHNUNG DER ERFINDUNG:
      Verfahren zur Aktivierung von denaturiertem Protein
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 41 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 1:
   AAAAAAGAAT TCTCATGATC GTGGGAGGCC AGGAATGCAA G 41
(2) ANGABEN ZU SEQ ID NO: 2:
   (i) SEQUENZKENNZEICHEN:
      (A) LÄNGE: 41 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 2:
   AAAAAAAAGC TTCATTACTT GGCCTTGGGC AAGCCCCTGG T 41
(2) ANGABEN ZU SEQ ID NO: 3:
   (i) SEQUENZKENNZEICHEN:
      (A) LÄNGE: 59 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 3:
   AAAAAAGAAT CCATTAAAGA GGAGAAATTA AAATGCGGAA GCTCTGCAGC CTGGACAAC 59
(2) ANGABEN ZU SEQ ID NO: 4:
   (i) SEQUENZKENNZEICHEN:
      (A) LÄNGE: 394 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 4:
   AGAATTCAAG AAGGAGATAT ACATATGTCA TCATCACATC CAATCTTCCA CAGGGGCGAG 60
   TTCTCGGTGT GTGACAGTGT CAGCGTGTGG GTTGGGGATA AGACCACCGC CACAGATATC 120
   AAGGGCAAGG AGGTGATGGT GTTGGGAGAG GTGAACATTA ACAACAGTGT ATTCAAACAG 180
   TACTTTTTTG AGACCAAGTG CCGGGACCCA AATCCCGTCG ACAGCGGGTG CCGGGGCATT 240
   GACTCAAAGC ACTGGAACTC ATATTGTACC ACGACTCACA CCTTTGTCAA GGCGCTGACC 300
   ATGGATGGCA AGCAGGCTGC CTGGCGGTTT ATCCGGATAG ATACGGCCTG TGTGTGTGTG 360
   CTCTCTAGAA AGGCTGTGAG ATGATAAAAG CTTG 394